# EUROPEAN PATENT SPECIFICATION

(11) **EP 4 471 279 B1**
(45) Date of publication and mention of the grant of the patent: **19.11.2025**
(21) Application number: 24179548.3
(22) Date of filing: 03.06.2024
(51) Int. Cl.: F16B 3/00

(54) **REVERSIBLE FASTENING MECHANISM FOR QUICK AND REVERSIBLE FIXING**
UMKEHRBARER BEFESTIGUNGSMECHANISMUS FÜR EINE SCHNELLE UND UMKEHRBARE BEFESTIGUNG
MÉCANISME DE FIXATION RÉVERSIBLE POUR UNE FIXATION RAPIDE ET RÉVERSIBLE

(30) Priority: 01.06.2023 PT 2023118694
(43) Date of publication of application: 04.12.2024
(73) Proprietor: António Cameira Eiras, Unipessoal Lda, 1250-247 Lisboa (PT)
(72) Inventor: CAMEIRA SILVA EIRAS, ANTONIO MANUEL, Lisboa (PT)
(74) Representative: Pereira da Cruz, Joao

(56) References cited:
- EP-A1- 0 338 082

## Description

### FIELD OF THE INVENTION

The present invention refers to the field of the mechanisms for fastening two or more structures, in a temporary or permanent manner.

### PRIOR ART

Fastening mechanisms are used to join, temporarily or permanently, two or more structures to each other.

When permanent fixation of structures is desired, we can opt for a mechanical device or a chemical union, using adhesive substances such as glues, or by welding, if applicable. For temporary connections, the fastening mechanisms to be used must be mechanical to allow repeated fastening and releasing procedures.

In any project, a fastening mechanism must be selected based on:
- the characteristics of the structures or technical device under design;
- the mechanical properties of the materials that make up the structures to be joined;
- the physical, chemical and environmental stresses to which the structures and the fixing device will be subjected, during the useful life of the structures or technical device under design.

The most commonly used mechanical fastening devices are screws. The use of screws, or sets of screws and nuts properly selected and suitable for a given project, allows for relatively quick, effective and long-lasting fixation.

However, in a temporary, reversible fixation that may be repetitive, this type of fixation is not suitable, due to the rapid deterioration of the support material in each repetition of the fastening and/or removal process, and the time it takes to fasten and/or remove these devices.

The proposed invention innovatively provides a solution for such problem.

From the document EP 0 338 082 A1 there is known a locking device.

### SUMMARY OF THE INVENTION

It is an object of the present invention a reversible fastening mechanism comprising an internal element (1), an external element (2) and a key (3), wherein the internal element (1) comprises a central cavity having a bottom wall, at least one spring (9) connected to said bottom wall and projecting to said cavity, and at least one stationary stop (7) positioned within said cavity; the external element (2) comprises a central hole, and at least one stationary stop (7) positioned within said central hole; the key (3) comprises an internal portion and an external portion (6), having said internal portion at least one internal lock (4) and at least one external lock (5); in which the internal element (1) is positioned adjacently to the external element (2) so that the central hole of the external element (2) is aligned with the central cavity of the internal element (1); the internal portion of the key (3) is configured to be inserted through the central hole of the external element (2) and through the central cavity of the internal element (1) to engage the spring (9) of the internal element (1); the external portion (6) of the key (3) is configured to allow a rotation movement of the key (3) so that the internal lock (4) of the key (3) engages the stationary stop (7) of the internal element (1) and the external lock (5) of the key (3) engages the stationary stop (7) of the external element (2).

The present invention concerns a quick and reversible fastening mechanism in which the internal element (1) and external element (2) are each installed in one of the structures to be joined, and a key (3), which ensures the rapid and precise fixation and release of these structures.

In a preferred aspect of the present invention, the internal element (1) and external element (2) are each one connected to a given structure that need to be fixed to one another, wherein the positional alignment of the internal element (1) with the external element (2) provide temporary contacts which are then secured using the key (3), which is inserted through the central hole and central cavity with minimal resistance and by rotating the external portion (6) of the key (3) allows the internal portion of the key (3) to rotate as well so that the internal lock (4) and external lock (5) may engage and be secured to the stationary stop (7)s of the internal element (1) and external element (2), respectively.

In addition, the spring (9) engages with the internal portion of the key (3), as it is inserted in the cavity of the internal element (1), thus exerting a constant force in the opposite direction, thereby pushing the internal lock (4) and external lock (5) against the stationary stop (7)s of the internal element (1) and of the external element (2), respectively, thus increasing the stability of the connection while also providing great speed to the fixing operations. In addition, if there is a need to quickly disrupt the connection, the rotation of the external portion (6) of the key (3) in the opposite direction will quickly release the internal element (1) and external element (2) from the key (3). At the same time, the mechanism is robust and allows repeated fixing and releasing operations while minimizing the deterioration of the mechanical elements involved in the connection.

Each reversible fastening mechanism can be manufactured according to the dimensional characteristics and materials that make up the structures to be joined, such as their dimensions (height, width and thickness), the physical, chemical and environmental stresses to which these structures will be predictably subject during their useful life, taking into account their mechanical resistance to rupture and/or their structural resistance to deformation and corrosion.

The main advantages of the object of the present invention are the quick and reversible mechanical fixing mechanism which is indicated for use in projects where the quickest and most precise fixation and release of structures or components is relevant, repeatedly or not, whether in processes of current use, whether for packaging or transport, or in maintenance and/or repair task; a greater flexibility in terms of the design, manufacture, functionality, logistics and maintenance of structures to use; and greater efficiency (increased safety, speed and precision) of the fixing and releasing processes of these structures.

The reversible fastening mechanism may be applicable in the automotive and other vehicle construction industries, in the aerospace industry, in the naval industry and in any other technical equipment subject to frequent maintenance, therefore may have several configurations and is not limited to any of its described embodiments.

### DESCRIPTION OF THE FIGURES

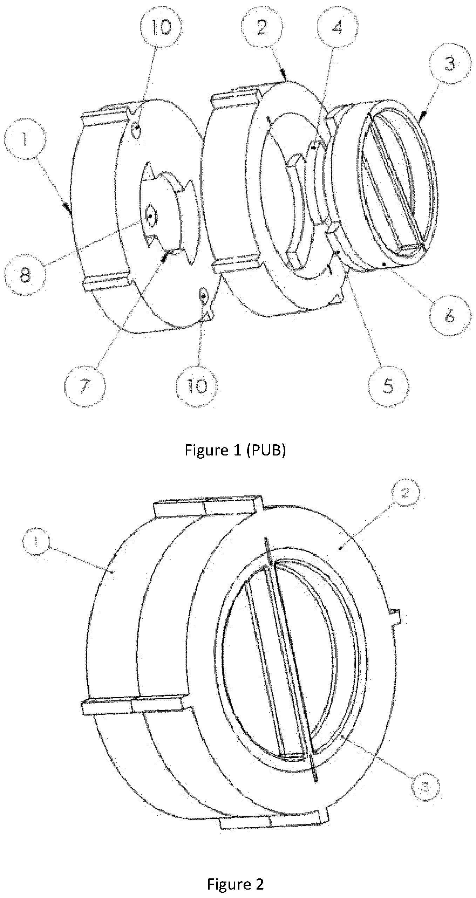

Figure 1 illustrates an exploded view from the exterior side of a preferred embodiment of the reversible fastening mechanism.
Figure 2 illustrates a perspective view from the exterior side of a preferred embodiment of the reversible fastening mechanism in an assembled configuration.

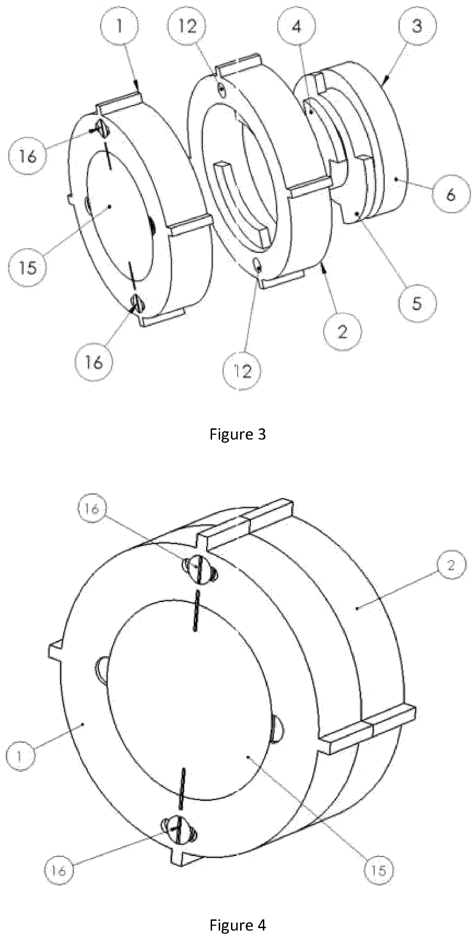

Figure 3 illustrates an exploded view from the interior side of a preferred embodiment of the reversible fastening mechanism.
Figure 4 illustrates a perspective view from the interior side of a preferred embodiment of the reversible fastening mechanism in an assembled configuration.

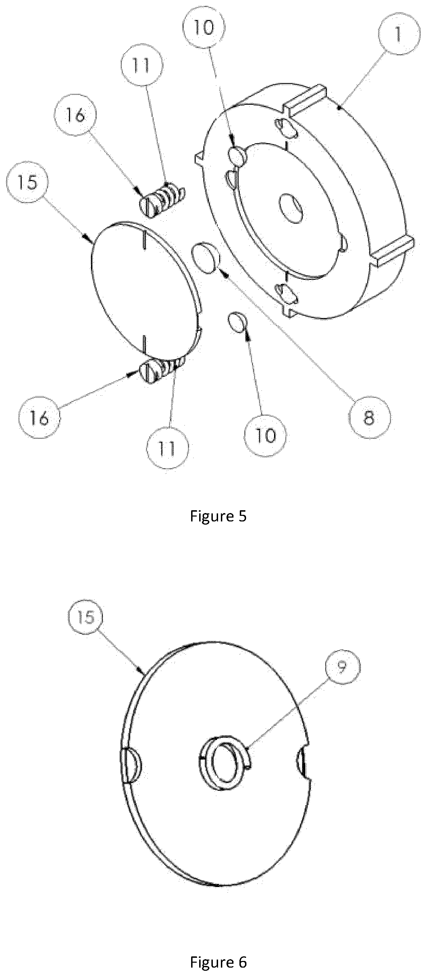

Figure 5 illustrates an exploded view from the interior side of the internal element (1) of the reversible fastening mechanism.
Figure 6 illustrates the interior side of the removable cap (15) with a central spring (9) (9).

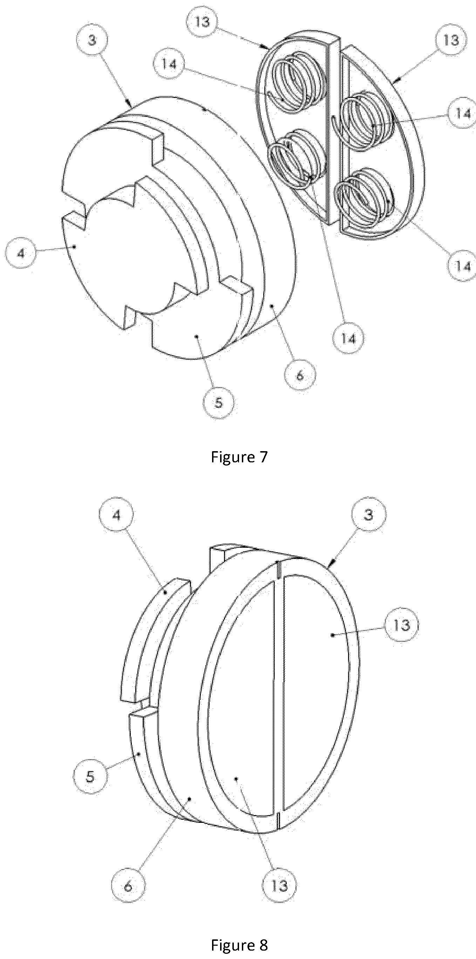

Figure 7 illustrates an exploded view from the interior side of the key (3) of the reversible fastening mechanism.
Figure 8 illustrates a perspective view from the exterior side of the key (3) of the fastening mechanism in an assembled configuration.

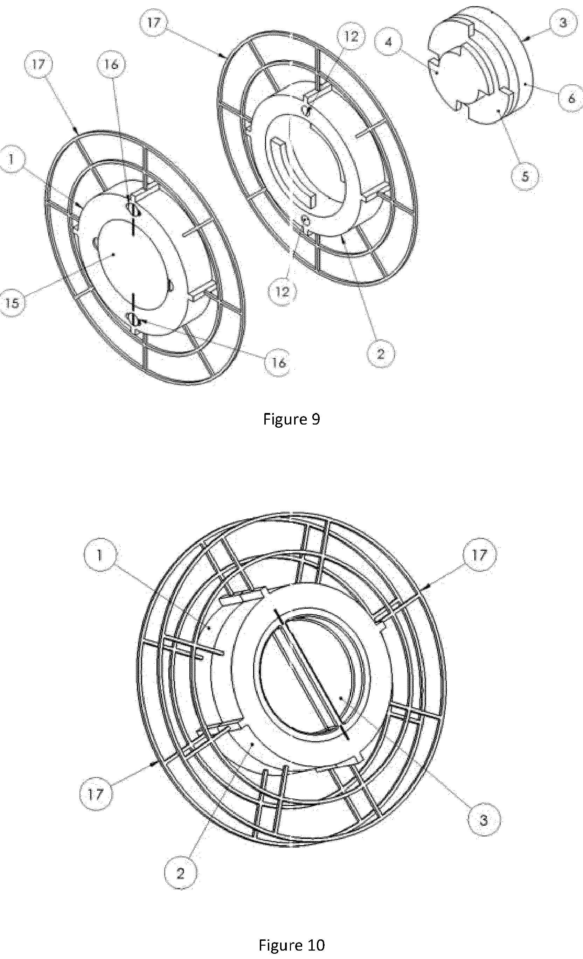

Figure 9 illustrates an exploded view from the interior side of a preferred embodiment of the reversible fastening mechanism, comprising two peripheral frame structure (17)s, being one peripheral frame structure (17) connected to the outer surface of the internal element (1), and being one peripheral frame structure (17) connected to the outer surface of the external element (2), wherein each peripheral frame structure (17) is positioned along a perpendicular plane to insertion position of the key (3).
Figure 10 illustrates a perspective view from the exterior side of another preferred embodiment of the reversible fastening mechanism comprising two peripheral frame structure (17)s, being the reversible fastening mechanism in an assembled configuration.

### DETAILED DESCRIPTION

The more general and advantageous configurations of the present invention are described in the Summary of the Invention. Such configurations are detailed below in accordance with other advantageous and/or preferred embodiments of implementation of the present invention.

In a preferred aspect of the present invention, the internal element (1) further comprises at least one fitting component (8) connected to the spring (9) and at least one peripheral fitting component (10); and the external element (2) comprises at least one peripheral fitting slot (12), so that the fitting component (8) connected to the spring (9) of the internal element (1) is adapted to engage the internal portion of the key (3) when the key (3) is fully inserted into the cavity of the internal element (1). Moreover, the peripheral fitting component (10) of the internal element (1) is configured to engage with the peripheral fitting slot (12) of the external element (2), thereby increasing the precision during the positioning and alignment of the internal element (1) and of the external element (2), prior to the insertion of the key (3). Another advantage is that the engagement between the peripheral fitting component (10) of the internal element (1) and the peripheral fitting slot (12) of the external element (2) does not interfere with the relative displacement of the internal element (1), external element (2) and key (3) to be fixed, either in the approach movement for fixing, or in the movement away to release the structures.

In a preferred embodiment of the present invention, the internal element (1) further comprises at least one peripheral cavity having a bottom wall to which a spring (11) is connected, being said spring (11) also connected to the peripheral fitting component (10), so that the spring (11) provides constant pressure upon the engagement between the peripheral fitting component (10) of the internal element (1) and the peripheral fitting slot (12) of the external element (2). The constant force exerted of the peripheral springs (11) promotes the engagement of the peripheral fitting component (10) of the internal element (1) with the peripheral fitting slot (12) of the external element (2), thereby increasing the stability of the assembly and also contributing to the stable engagement of the stationary stop (7)s of the internal element (1) and external element (2) with the internal and external locks (5) of the key (3). Another advantage of the peripheral springs (11) is the increase of the speed of assembly, wherein the peripheral fitting components (10) are easily driven towards the corresponding fitting slot by the natural expansion tendency of a spring (11) being compressed when the internal element (1) and the external element (2) are positioned closer together and aligned, prior to the insertion of the key (3).

In another preferred embodiment of the present invention, the bottom wall of the central cavity and the bottom wall of the peripheral cavity of the internal element (1) each comprise a removable cap (16). The advantage to have the bottom of the central cavity is that it facilitates the access to the cavity of the internal element (1) in order to perform maintenance operations, such as repairing of the central spring (9) or of the fitting component (8), or even replacement of the central spring (9) or fitting component (8). Similarly, the peripheral cavity of the internal element (1) may also comprise a removable cap (16) to allow maintenance operations to repair or replace the peripheral springs (11), if necessary.

Preferably, the internal element (1) of the reversible fastening mechanism may comprise at least two peripheral fitting components (10), each one configured to engage with one peripheral fitting slot (12) of the external element (2). In addition, the internal element (1) of the reversible fastening mechanism may comprise at least two peripheral cavities, each peripheral cavity having a bottom wall and comprising one spring (11) connected to one peripheral fitting component (10), wherein the bottom wall of each peripheral cavity of the internal element (1) may comprise a removable cap (16).

In a more preferred embodiment of the present invention, the shape of the fitting component (8) and the shape of the peripheral fitting components (10) is semispherical, in which the peripheral fitting slot (12) of the external element (2) may have a complementary shape to the semispherical peripheral fitting component (10) of the internal element (1), thereby increasing the stability of the connection. Moreover, the internal portion of the key (3) may comprise a fitting slot adapted to engage with the fitting component (8) connected to the spring (9) of the central cavity, having said fitting slot a complementary shape to the semispherical fitting component (8) of the internal element (1). By fitting complementary shapes, in which the fitting component (8) has a semispherical shape, the precision of the coupling of the two complementary parts is increased, as well as the speed of said coupling. In addition, the semispherical shape minimizes the deterioration of the coupled parts during repeated fixing and releasing operations.

In another preferred embodiment of the present invention, the external portion (6) of the key (3) comprises at least two cavities, each cavity comprising a bottom wall and at least one spring (14) attached to said bottom wall and to a button cap (13). The advantage of the cavities comprised in the external portion (6) of the key (3) is to improve the grip to facilitate the rotation of the key (3), by having two button cap (13)s which collapse into the cavity, allowing the user to grip the inside walls of the cavities of the external portion (6) of the key (3) to manipulate the key (3).

In a more advantageous aspect of the present invention, each button cap (13) covers the cavity completely and is leveled with the surface of the external portion (6) of the key (3), being each button cap (13) configured to allow being pushed inside a cavity against the force exerted by the spring (14), in which the button cap (13) returns to a leveled position with the surface of the external portion (6) of the key (3) in the absence of forces applied to the button cap (13). By covering the cavity and being leveled with the surface of the external portion (6) of the key (3), the aerodynamic performance or the reversible fastening mechanism is improved while maintaining the functionality of improving the grip of the external portion (6) of the key (3) to improve the handling of the key (3), thus allowing the application of the reversible fastening mechanism to minimalistic structures or systems in which aerodynamic performance is important.

In another embodiment of the present invention, the internal element (1) and the external element (2) each comprise one peripheral frame structure (17), being said peripheral frame structure (17) connected to the lateral surface of the internal element (1) and/or the lateral surface of the external element (2). The incorporation of peripheral frame structure (17) to the internal element (1) and external element (2) allows a more precise and stable application of the reversible fastening mechanism into structures made of composite materials, in which two composite materials can be fixed together by first being connected to each peripheral frame structure (17) to then be secured by the engagement between the internal element (1), external element (2) and the key (3). Preferably, the peripheral frame structure (17) is positioned along a perpendicular plane to insertion position of the key (3).

To facilitate the rotation of the key (3), the internal portion and the external portion (6) of the key (3) preferably comprise a cylindrical shaft.

The reversible fastening mechanism may be manufactured using several materials, such as metals, hard plastics, wood and composite materials.

The method to use the reversible fastening mechanism comprise the following steps:
- For fixing at least two structures together, the structures are brought close together, wherein one structure comprises an internal element (1) of the reversible fastening mechanism and the other structure comprises the external element (2) of the reversible fastening mechanism;
- The respective internal element (1) and the external element (2) are brought close together and are aligned;
- The peripheral fitting components (10) of the internal element (1) are coupled to the respective peripheral fitting slot (12)s of the external element (2), securing the internal element (1) and the external element (2) in the proper position;
- A key (3) is inserted, being the internal portion of the key (3) inserted fully through the central hole of the external element (2) until the fitting slot of the internal portion of the key (3) is coupled to the fitting element connected to the spring (9) of the cavity of the internal element (1), being the key (3) under pressure to overcome the resistance of the spring (9);
- The key (3) is rotated by a maximum of a quarter turn, in a counterclockwise direction, until the internal lock (4) and the external lock (5) of the key (3) contact with the stationary stop (7) of the internal element (1) and with the stationary stop (7) of the external element (2);
- To release the fixed at least two structures, the key (3) is pressed to overcome the resistance of the spring (9) of the central cavity of the internal element (1) and is rotated a maximum of a quarter turn, in a clockwise direction;
- The internal lock (4) and external lock (5) of the key (3) are released simultaneously from the stationary stop (7)s of the internal element (1) and of the external element (2);
- The key (3) is removed from the internal element (1) and from the external element (2);
- The internal element (1) and external element (2) are separated by a sliding movement or by axial or perpendicular separation, in which the peripheral fittings are released from the peripheral fitting slots (12).

Of course, the preferred embodiments shown above are combinable, in different possible configurations, being the present invention not limited to the embodiments previously described.

## Claims

1. A reversible fastening mechanism comprising an internal element (1), an external element (2) and a key (3), wherein:
the internal element (1) comprises a central cavity having a bottom wall, at least one spring (9) connected to said bottom wall and projecting to said cavity, and at least one stationary stop (7) positioned within said cavity;
the external element (2) comprises a central hole, and at least one stationary stop (7) positioned within said central hole;
the key (3) comprises an internal portion and an external portion (6), having said internal portion at least one internal lock (4) and at least one external lock (5);
in which the internal element (1) is positioned adjacently to the external element (2) so that the central hole of the external element (2) is aligned with the central cavity of the internal element (1); the internal portion of the key (3) is configured to be inserted through the central hole of the external element (2) and through the central cavity of the internal element (1) to engage the spring (9) of the internal element (1); the external portion (6) of the key (3) is configured to allow a rotation movement of the key (3) so that the internal lock (4) of the key (3) engages the stationary stop (7) of the internal element (1) and the external lock (5) of the key (3) engages the stationary stop (7) of the external element (2).

2. A reversible fastening mechanism according to claim 1 wherein:
the internal element (1) further comprises at least one fitting component (8) connected to the spring (9) and at least one peripheral fitting component (10); the external element (2) comprises at least one peripheral fitting slot (12);
in which the fitting component (8) connected to the spring (9) of the internal element (1) is adapted to engage the internal portion of the key (3); and the peripheral fitting component (10) of the internal element (1) is configured to engage with the peripheral fitting slot (12) of the external element (2).

3. A reversible fastening mechanism according to claim 2 wherein the internal element (1) comprises at least one peripheral cavity having a bottom wall and comprising a spring (11) connected to the peripheral fitting component (10) to provide constant pressure upon the engagement between the peripheral fitting component (10) of the internal element (1) and the peripheral fitting slot (12) of the external element (2).

4. A reversible fastening mechanism according to any of the preceding claims wherein the bottom wall of the central cavity and the bottom wall of the peripheral cavity of the internal element (1) each comprise a removable cap (15/16).

5. A reversible fastening mechanism according to any of the preceding claims wherein the external portion (6) of the key (3) comprises at least two cavities, each cavity comprising a bottom wall and at least one spring (14) attached to said bottom wall and to a button cap (13), in which the button cap (13) covers the cavity completely and is leveled with the surface of the external portion (6) of the key (3), being each button cap (13) configured to allow being pushed inside a cavity against the force exerted by the spring (14).

6. A reversible fastening mechanism according to any of the preceding claims wherein the internal element (1) and the external element (2) each comprise one peripheral frame structure (17), being said peripheral frame structure (17) connected to the lateral surface of the internal element (1) and/or the lateral surface of the external element (2) and being said peripheral frame structure (17) positioned along a perpendicular plane to insertion position of the key (3).

7. A reversible fastening mechanism according to claim 2 wherein the fitting component (8) and the peripheral fitting component (10) of the internal element (1) comprise a semispherical shape.

8. A reversible fastening mechanism according to claim 2 wherein the internal portion of the key (3) comprises a fitting slot adapted to engage with the fitting component (8) connected to the spring (9) of the central cavity of the internal element (1).

9. A reversible fastening mechanism according to claim 7 - 8 wherein the fitting slot of the internal portion of the key (3) comprises a complementary shape to the shape of the fitting component (8) of the internal element (1), and the peripheral fitting slot (12) of the external element (2) comprises a complementary shape to the shape of the fitting component (8) of the internal element (1).

10. A reversible fastening mechanism according to any of the preceding claims wherein the internal portion and the external portion (6) of the key (3) comprise a cylindrical shaft.

11. A reversible fastening mechanism according to any of the preceding claims wherein the internal element (1), the external element (2) and the key (3) are made of a one or more materials selected from the group: metal, hard plastics, wood and composite materials.

12. A method to use the reversible fastening mechanism according to the claims 1 - 11 comprising the following steps:
- For fixing at least two structures together, the structures are brought close together, wherein one structure comprises an internal element (1) of the reversible fastening mechanism and the other structure comprises the external element (2) of the reversible fastening mechanism;
- The respective internal element (1) and the external element (2) are brought close together and are aligned;
- The peripheral fitting components (10) of the internal element (1) are coupled to the respective peripheral fitting slot (12)s of the external element (2), securing the internal element (1) and the external element (2) in the proper position;
- A key (3) is inserted, being the internal portion of the key (3) inserted fully through the central hole of the external element (2) until the fitting slot of the internal portion of the key (3) is coupled to the fitting element connected to the spring (9) of the cavity of the internal element (1), being the key (3) under pressure to overcome the resistance of the spring (9);
- The key (3) is rotated by a maximum of a quarter turn, in a counterclockwise direction, until the internal lock (4) and the external lock (5) of the key (3) contact with the stationary stop (7) of the internal element (1) and with the stationary stop (7) of the external element (2);
- To release the fixed at least two structures, the key (3) is pressed to overcome the resistance of the spring (9) of the central cavity of the internal element (1) and is rotated a maximum of a quarter turn, in a clockwise direction;
- The internal lock (4) and external lock (5) of the key (3) are released simultaneously from the stationary stop (7)s of the internal element (1) and of the external element (2);
- The key (3) is removed from the internal element (1) and from the external element (2);
- The internal element (1) and external element (2) are separated by a sliding movement or by axial or perpendicular separation, in which the peripheral fittings are released from the peripheral fitting slots (12).

## Patentansprüche

1. Umkehrbarer Befestigungsmechanismus mit einem inneren Element (1), einem äußeren Element (2) und einem Schlüssel (3), wobei:
das innere Element (1) einen zentralen Hohlraum mit einer Bodenwand, mindestens eine Feder (9), die mit der Bodenwand verbunden ist und in den Hohlraum hineinragt, und mindestens einen stationären Anschlag (7) aufweist, der in dem Hohlraum angeordnet ist;
das äußere Element (2) ein zentrales Loch und mindestens einen stationären Anschlag (7) aufweist, der in dem zentralen Loch angeordnet ist;
der Schlüssel (3) einen inneren Teil und einen äußeren Teil (6) umfasst, wobei der innere Teil mindestens eine innere Verriegelung (4) und mindestens eine äußere Verriegelung (5) aufweist;
wobei das innere Element (1) an das äußere Element (2) angrenzend positioniert ist, so dass das zentrale Loch des äußeren Elements (2) mit dem zentralen Hohlraum des inneren Elements (1) ausgerichtet ist; der innere Teil des Schlüssels (3) so konfiguriert ist, dass er durch das zentrale Loch des äußeren Elements (2) und durch den zentralen Hohlraum des inneren Elements (1) eingeführt wird, um mit der Feder (9) des inneren Elements (1) in Eingriff zu kommen; der äußere Teil (6) des Schlüssels (3) so konfiguriert ist, dass er eine Drehbewegung des Schlüssels (3) ermöglicht, so dass die innere Verriegelung (4) des Schlüssels (3) in den stationären Anschlag (7) des inneren Elements (1) und die äußere Verriegelung (5) des Schlüssels (3) in den stationären Anschlag (7) des äußeren Elements (2) eingreift.

2. Umkehrbarer Befestigungsmechanismus nach Anspruch 1, wobei:
das innere Element (1) ferner mindestens ein mit der Feder (9) verbundene Passkomponente (8) und mindestens ein periphere Passkomponente (10) aufweist;
das äußere Element (2) mindestens einen peripheren Passschlitz (12) aufweist; wobei die mit der Feder (9) des inneren Elements (1) verbundene Passkomponente (8) so geeignet ist, dass sie in den inneren Teil des Schlüssels (3) eingreift; und die periphere Passkomponente (10) des inneren Elements (1) so konfiguriert ist, dass sie in den peripheren Passschlitz (12) des äußeren Elements (2) eingreift.

3. Umkehrbarer Befestigungsmechanismus nach Anspruch 2, wobei das innere Element (1) mindestens einen peripheren Hohlraum mit einer Bodenwand und einer Feder (11) aufweist, die mit der peripheren Passkomponente (10) verbunden ist, um beim Eingriff zwischen der peripheren Passkomponente (10) des inneren Elements (1) und dem peripheren Passschlitz (12) des äußeren Elements (2) einen konstanten Druck auszuüben

4. Umkehrbarer Befestigungsmechanismus nach einem der vorhergehenden Ansprüche, wobei die Bodenwand des zentralen Hohlraums und die Bodenwand des peripheren Hohlraums des inneren Elements (1) jeweils eine abnehmbare Kappe (15/16) aufweisen.

5. Umkehrbarer Befestigungsmechanismus nach einem der vorhergehenden Ansprüche, wobei der äußere Teil (6) des Schlüssels (3) mindestens zwei Hohlräume aufweist, wobei jeder Hohlraum eine Bodenwand und mindestens eine Feder (14) umfasst, die an der Bodenwand und an einer Knopfkappe (13) befestigt ist, wobei die Knopfkappe (13) den Hohlraum vollständig abdeckt und mit der Oberfläche des äußeren Teils (6) des Schlüssels (3) bündig ist, wobei jede Knopfkappe (13) so konfiguriert ist, dass sie gegen die von der Feder (14) ausgeübte Kraft in einen Hohlraum gedrückt werden kann.

6. Umkehrbarer Befestigungsmechanismus nach einem der vorhergehenden Ansprüche, wobei das innere Element (1) und das äußere Element (2) jeweils eine periphere Rahmenstruktur (17) aufweisen, wobei die periphere Rahmenstruktur (17) mit der Seitenfläche des inneren Elements (1) und/oder der Seitenfläche des äußeren Elements (2) verbunden ist und wobei die periphere Rahmenstruktur (17) entlang einer senkrechten Ebene zur Einführposition des Schlüssels (3) angeordnet ist.

7. Umkehrbarer Befestigungsmechanismus nach Anspruch 2, wobei die Passkomponente (8) und die periphere Passkomponente (10) des inneren Elements (1) eine halbkugelförmige Form aufweisen.

8. Umkehrbarer Befestigungsmechanismus nach Anspruch 2, wobei der innere Teil des Schlüssels (3) einen Passschlitz aufweist, der dazu geeignet ist, mit der Passkomponente (8) in Eingriff zu kommen, die mit der Feder (9) des zentralen Hohlraums des inneren Elements (1) verbunden ist.

9. Umkehrbarer Befestigungsmechanismus nach Anspruch 7-8, wobei der Passschlitz des inneren Teils des Schlüssels (3) eine zur Form der Passkomponente (8) des inneren Elements (1) komplementäre Form aufweist, und der periphere Passschlitz (12) des äußeren Elements (2) eine zur Form der Passkomponente (8) des inneren Elements (1) komplementäre Form aufweist.

10. Umkehrbarer Befestigungsmechanismus nach einem der vorhergehenden Ansprüche, wobei der innere Teil und der äußere Teil (6) des Schlüssels (3) einen zylindrischen Schaft aufweisen.

11. Umkehrbarer Befestigungsmechanismus nach einem der vorhergehenden Ansprüche, wobei das innere Element (1), das äußere Element (2) und der Schlüssel (3) aus einem oder mehreren Materialien hergestellt sind, die aus der Gruppe Metall, harter Kunststoff, Holz und Verbundmaterialien ausgewählt sind.

12. Verfahren zur Verwendung des umkehrbarer Befestigungsmechanismus nach Anspruch 1-11, umfassend die folgenden Schritte:
- Zum Befestigen von mindestens zwei Strukturen werden die Strukturen aneinander angenähert, wobei eine Struktur ein Innere Element (1) des umkehrbarer Befestigungsmechanismus und die andere Struktur des äußeren Element (2) des umkehrbarer Befestigungsmechanismus umfasst;
- Das jeweilige innere Element (1) und das äußere Element (2) werden nahe zueinander gebracht und ausgerichtet;
- Die peripheren Passkomponenten (10) des inneren Elements (1) werden mit den entsprechenden peripheren Passschlitzen (12) des äußeren Elements (2) gekoppelt, wodurch das innere Element (1) und das äußere Element (2) in der richtigen Position gesichert werden;
- Ein Schlüssel (3) wird eingeführt, wobei der innere Teil des Schlüssels (3) vollständig durch das zentrale Loch des äußeren Elements (2) eingeführt wird, bis der Passschlitz des inneren Teils des Schlüssels (3) mit dem mit der Feder (9) des Hohlraums des inneren Elements (1) verbundenen Passkomponente gekoppelt ist, wobei der Schlüssel (3) unter Druck steht, um den Widerstand der Feder (9) zu überwinden;
- Der Schlüssel (3) wird um maximal eine Vierteldrehung gegen den Uhrzeigersinn gedreht, bis die innere Verriegelung (4) und die äußere Verriegelung (5) des Schlüssels (3) mit dem stationären Anschlag (7) des inneren Elements (1) und mit dem stationären Anschlag (7) des äußeren Elements (2) in Berührung kommen;
- Um die mindestens zwei feststehenden Strukturen zu lösen, wird der Schlüssel (3) gedrückt, um den Widerstand der Feder (9) des zentralen Hohlraums des innere Elements (1) zu überwinden, und maximal eine Vierteldrehung im Uhrzeigersinn gedreht;
- Die innere Verriegelung (4) und die äußere Verriegelung (5) des Schlüssels (3) werden gleichzeitig von den stationären Anschlägen (7) des inneren Elements (1) und des äußeren Elements (2) gelöst;
- Der Schlüssel (3) wird aus dem innere Element (1) und aus dem äußeren Element (2) entfernt;
- Das innere Element (1) und das äußeren Element (2) werden durch eine Gleitbewegung oder durch axiale oder senkrechte Trennung getrennt, wobei die peripheren Passkomponenten aus den peripheren Passschlitzen (12) gelöst werden.

## Revendications

1. Mécanisme de fixation réversible comprenant un élément interne (1), un élément externe (2) et une clé (3), dans lequel :
l'élément interne (1) comprend une cavité centrale ayant une paroi inférieure, au moins un ressort (9) relié à ladite paroi inférieure et faisant saillie vers ladite cavité, et au moins une butée fixe (7) positionnée à l'intérieur de ladite cavité ;
l'élément externe (2) comprend un trou central et au moins une butée fixe (7) positionnée à l'intérieur dudit trou central ;
la clé (3) comprend une partie interne et une partie externe (6), ladite partie interne comportant au moins un verrou interne (4) et au moins un verrou externe (5) ;
où l'élément interne (1) est positionné de manière adjacente à l'élément externe (2), de sorte que l'orifice central de l'élément externe (2) soit aligné avec la cavité centrale de l'élément interne (1) ; la partie interne de la clé (3) est configurée pour être insérée à travers l'orifice central de l'élément externe (2) et à travers la cavité centrale de l'élément interne (1) afin de venir en prise avec le ressort (9) de l'élément interne (1) ; la partie externe (6) de la clé (3) est configurée pour permettre un mouvement de rotation de la clé (3), de sorte que le verrou interne (4) de la clé (3) vienne en prise avec la butée fixe (7) de l'élément interne (1) et que le verrou externe (5) de la clé (3) vienne en prise avec la butée fixe (7) de l'élément externe (2).

2. Mécanisme de fixation réversible selon la revendication 1, dans lequel :
l'élément interne (1) comprend en outre au moins un composant d'ajustement (8) relié au ressort (9) et au moins un composant d'ajustement périphérique (10) ;
l'élément externe (2) comprend au moins une fente d'ajustement périphérique (12) ;
où le composant d'ajustement (8) relié au ressort (9) de l'élément interne (1) est adapté pour venir en prise avec la partie interne de la clé (3) ; et le composant d'ajustement périphérique (10) de l'élément interne (1) est configuré pour venir en prise avec la fente d'ajustement périphérique (12) de l'élément externe (2).

3. Mécanisme de fixation réversible selon la revendication 2, dans lequel l'élément interne (1) comprend au moins une cavité périphérique ayant une paroi inférieure et comprenant un ressort (11) relié au composant d'ajustement périphérique (10) afin d'exercer une pression constante lors de l'engagement entre le composant d'ajustement périphérique (10) de l'élément interne (1) et la fente d'ajustement périphérique (12) de l'élément externe (2).

4. Mécanisme de fixation réversible selon l'une quelconque des revendications précédentes, dans lequel la paroi inférieure de la cavité centrale et la paroi inférieure de la cavité périphérique de l'élément interne (1) comprennent chacune un capuchon amovible (15/16).

5. Mécanisme de fixation réversible selon l'une quelconque des revendications précédentes, dans lequel la partie externe (6) de la clé (3) comprend au moins deux cavités, chaque cavité comprenant une paroi inférieure et au moins un ressort (14) fixé à ladite paroi inférieure et à un capuchon de bouton (13), où le capuchon de bouton (13) couvre complètement la cavité et est de niveau avec la surface de la partie externe (6) de la clé (3), chaque capuchon de bouton (13) étant configuré pour pouvoir être enfoncé dans une cavité contre la force exercée par le ressort (14).

6. Mécanisme de fixation réversible selon l'une quelconque des revendications précédentes, dans lequel l'élément interne (1) et l'élément externe (2) comprennent chacun une structure de cadre périphérique (17), ladite structure de cadre périphérique (17) étant reliée à la surface latérale de l'élément interne (1) et/ou à la surface latérale de l'élément externe (2) et ladite structure de cadre périphérique (17) étant positionnée le long d'un plan perpendiculaire à la position d'insertion de la clé (3).

7. Mécanisme de fixation réversible selon la revendication 2, dans lequel le composant d'ajustement (8) et le composant d'ajustement périphérique (10) de l'élément interne (1) présentent une forme hémisphérique.

8. Mécanisme de fixation réversible selon la revendication 2, dans lequel la partie interne de la clé (3) comprend une fente d'ajustement adaptée pour venir en prise avec le composant d'ajustement (8) relié au ressort (9) de la cavité centrale de l'élément interne (1).

9. Mécanisme de fixation réversible selon les revendications 7-8, dans lequel la fente d'ajustement de la partie interne de la clé (3) présente une forme complémentaire à la forme du composant d'ajustement (8) de l'élément interne (1), et la fente d'ajustement périphérique (12) de l'élément externe (2) présente une forme complémentaire à la forme du composant d'ajustement (8) de l'élément interne (1).

10. Mécanisme de fixation réversible selon l'une quelconque des revendications précédentes, dans lequel la partie interne et la partie externe (6) de la clé (3) comprennent un arbre cylindrique.

11. Mécanisme de fixation réversible selon l'une quelconque des revendications précédentes, dans lequel l'élément interne (1), l'élément externe (2) et la clé (3) sont réalisés en un ou plusieurs matériaux choisis parmi le groupe comprenant : métal, plastiques durs, bois et matériaux composites.

12. Procédé d'utilisation du mécanisme de fixation réversible selon les revendications 1 à 11, comprenant les étapes suivantes :
- Pour fixer ensemble au moins deux structures, les structures sont rapprochées, une structure comprenant un élément interne (1) du mécanisme de fixation réversible et l'autre structure comprenant l'élément externe (2) du mécanisme de fixation réversible ;
- L'élément interne (1) et l'élément externe (2) respectifs sont rapprochés et alignés ;
- Les composants d'ajustement périphériques (10) de l'élément interne (1) sont couplés aux fentes d'ajustement périphériques respectives (12) de l'élément externe (2), fixant ainsi l'élément interne (1) et l'élément externe (2) dans la position correcte ;
- Une clé (3) est insérée, la partie interne de la clé (3) étant insérée complètement à travers l'orifice central de l'élément externe (2) jusqu'à ce que la fente d'ajustement de la partie interne de la clé (3) soit couplée à l'élément d'ajustement relié au ressort (9) de la cavité de l'élément interne (1), la clé (3) étant sous pression pour surmonter la résistance du ressort (9) ;
- La clé (3) est tournée d'un quart de tour maximum, dans le sens antihoraire, jusqu'à ce que le verrou interne (4) et le verrou externe (5) de la clé (3) entrent en contact avec la butée fixe (7) de l'élément interne (1) et avec la butée fixe (7) de l'élément externe (2) ;
- Pour libérer les au moins deux structures fixes, la clé (3) est pressée pour surmonter la résistance du ressort (9) de la cavité centrale de l'élément interne (1) et est tournée d'un quart de tour maximum, dans le sens horaire ;
- Le verrou interne (4) et le verrou externe (5) de la clé (3) sont libérés simultanément des butées fixes (7) de l'élément interne (1) et de l'élément externe (2) ;
- La clé (3) est retirée de l'élément interne (1) et de l'élément externe (2) ;
- L'élément interne (1) et l'élément externe (2) sont séparés par un mouvement coulissant ou par une séparation axiale ou perpendiculaire, où les composants d'ajustement périphériques sont libérés des fentes d'ajustement périphériques (12).
